# EUROPEAN PATENT APPLICATION

(11) **EP 2 314 328 A2**
(43) Date of publication of application: **27.04.2011**
(21) Application number: 10251852.9
(22) Date of filing: 26.10.2010
(51) Int. Cl.: A61L 31/00, A61L 31/08

(54) **Reactive surgical implant**

(30) Priority: 26.10.2009 US 605538
(71) Applicant: Tyco Healthcare Group LP, New Haven, CT 06511 (US)
(72) Inventor: Abuzaina, Ferass, Shelton, CT 06484 (US); Hotter, Joseph, Middletown, CT 06457 (US); Stopek, Joshua B., Guilford, CT 06437 (US)
(74) Representative: Soames, Candida Jane

(57) **Abstract**

Biocompatible implants include a polymer substrate and a reactive component selectively applied to the substrate. The reactive component in combination with the substrate creates crosslinked regions on a surface of the substrate. The crosslinked regions may provide a visual aid and/or stiffening element to the implant.

## Description

### CROSS-REFERENCE TO RELATED APPLICATIONS

This application is a continuation-in-part of U.S. Patent Application No. 12/548,524, filed August 27, 2009, which claims the benefit of and priority to U.S. Provisional Application No. 61/100,392, filed on September 26, 2008, the entire disclosures of each of which are incorporated by reference herein.

### TECHNICAL FIELD

The present disclosure relates to implants that include regions selectively crosslinked with a reactive component and, more particularly, to a surgical implant which crosslinks to a tissue surface in situ, provides a visual indicator on the surface of the surgical implant, and/or provides stiffened regions to the implant for ease of manipulation during a surgical procedure.

### BACKGROUND

The use of medical devices, and more specifically, implants, are known. However, the implants, once placed in situ, may dislocate or migrate resulting in damage to the surrounding tissue, or device failure depending on the type and location of the implant. Therefore, implants such as meshes, are frequently secured to tissue during surgery using surgical tacking devices or fasteners, such as staples, clips, tacks, sutures, and the like.

For example, a soft pliant mesh is typically used for surgical repair of body tissues and secured to the tissue with a surgical fastener. The flexible nature of the mesh, however, may make it hard to use, handle, and unfold, especially in a wet environment. Kinks and folds in the mesh are not acceptable as they can create dead-spaces allowing seromas and fistulas to develop which can get infected, cause implant failure, and may cause a disease state to recur.

Laparoscopic and endoscopic surgical procedures are being used for delivery of implants to a desired body location. In laparoscopic procedures, surgery is performed in the interior of the abdomen through a small incision. Similarly, in endoscopic procedures, surgery is performed in any hollow viscus of the body through narrow endoscopic tubes inserted through small entrance wounds in the skin.

Accordingly, an implant, such as a surgical mesh, may have to be rolled or folded in order to be placed through a laparoscopic port, such as a cannula or narrow endoscopic tube, for placement within the body. After insertion, the surgeon may have to take time and have the skill to unfold and properly place the mesh. Thus, a stiffer mesh may be easier to unfold by surgeons to the repair site as it may more readily spring back to its original shape during a laparoscopic procedure after insertion through a laparoscopic port.

Moreover, color identification means on the mesh may aid the surgeon in properly sizing, placing, and positioning the mesh during a surgical procedure, such as a laparoscopic or endoscopic procedure.

While current surgical implants and surgical methods perform satisfactorily, it would be advantageous to provide an implant with enhanced visualization and placement during a surgical procedure.

### SUMMARY

The present disclosure provides medical devices, in embodiments meshes, methods for their production, as well as methods for using same in surgical procedures. In embodiments, a medical device of the present disclosure may include a substrate including at least one synthetic component; at least one natural material on at least a portion of a surface of the substrate; and a reactive component selectively applied to at least a portion of the medical device, wherein the reactive component reacts with the at least one natural material to form at least one crosslinked region on the surface of the substrate.

A method of the present disclosure may include, in embodiments, a method for increasing the stiffness of a mesh. Such a method may include providing a substrate including at least one synthetic component in combination with at least one natural material including amine groups on at least a portion of a surface of the substrate; contacting the substrate with a genipin solution; and allowing the natural material and genipin to crosslink thereby creating localized crosslinked regions on the surface of the substrate, wherein the localized crosslinked regions increase stiffness of the mesh.

In other embodiments, a mesh of the present disclosure may include a polyester substrate; a collagen film on at least a portion of a surface of the polyester substrate; and a reactive component including genipin selectively applied to at least a portion of the mesh, wherein the genipin crosslinks the collagen, thereby increasing stiffness of the mesh.

### BRIEF DESCRIPTION OF THE DRAWINGS

The illustrative embodiments described herein will become more readily apparent from the following description, reference being made to the accompanying drawings in which:

FIG. 1 shows a coated reactive implant in accordance with one embodiment of the present disclosure;

FIG. 2 shows an alternate embodiment of a reactive implant in accordance with the present disclosure;

FIG. 3 illustrates an implant including selectively crosslinked regions in accordance with one embodiment of the present disclosure;

FIG. 4 illustrates an implant in accordance with another embodiment of the present disclosure;

FIG. 5 illustrates an implant in accordance with yet another embodiment of the present disclosure; and

FIG. 6 illustrates an implant in accordance with another embodiment of the present disclosure.

### DETAILED DESCRIPTION

Medical devices described herein include a substrate used in combination with a reactive component creating a reactive implant. The reactive implant self-fixates to an amine containing surface such as tissue, by crosslinking or chemically bonding with reactive groups present on the tissue surface. The term "chemical bonding" as used herein refers to all types of chemical bonding including covalent bonding, crosslinking, ionic bonding, and the like. Also as used herein, the term "device" and "implant" are interchangeable, however, once the device or implant contacts a reactive component, a "reactive device" or "reactive implant" is created. Also used herein, the term "reactive solution" includes a solution containing a reactive component.

Medical devices of the present disclosure can be made from natural materials, biodegradable materials, non-biodegradable materials, and combinations thereof. Suitable synthetic biodegradable materials include polymers such as those derived from lactide; glycolide; caprolactone; valerolactone; carbonates (e.g., trimethylene carbonate, tetramethylene carbonate, and the like); dioxanones (e.g., 1,4-dioxanone); δ-valerolactone; 1,dioxepanones (e.g., 1,4-dioxepan-2-one and 1,5-dioxepan-2-one); ethylene glycols; ethylene oxides; esteramides; γ-hydroxyvalerates; β-hydroxypropionates; alpha-hydroxy acids; hydroxybutyrates; poly (ortho esters); hydroxy alkanoates; tyrosine carbonates; poly(imide carbonates); poly(imino carbonates) such as poly (bisphenol A-iminocarbonate) and poly (hydroquinone-iminocarbonate); polyurethanes; polyanhydrides; polymer drugs (e.g., polydiflunisol, polyaspirin, and protein therapeutics); and copolymers and combinations thereof.

Suitable natural biodegradable polymers include proteins such as collagen, gelatin, albumin, casein, and poly (amino acids); polysaccharides such as cellulose, dextran, chitin, alginate, fucans, carboxymethyl cellulose and glycosaminoglycans; hyaluronic acid; gut; and copolymers and combinations thereof.

More specifically, collagen as defined herein includes natural or derived collagen (including animal or bacterial recombinant) and collagen derivatives. Collagen implants may include collagen derived from human, animal, bacterial, or recombinant origin. Some non-limiting examples include type I porcine or bovine collagen, type I, II, III, or IV human collagen, or combinations thereof. In one example, collagen includes the collagen construct in a PARIETEX™ Composite Mesh (PCO), commercially available from Tyco Healthcare Group, d\b\a, Covidien, North Haven, CT.

Collagen can be modified by using any method within the purview of those skilled in the art to provide pendant portions of the collagen with moieties which are capable of covalently bonding with the reactive chemical groups of a glycosaminoglycan. Examples of such pendant moieties include aldehydes, sulfones, vinylsulfones, isocyanates, and acid anhydrides. In addition, electrophilic groups such as -CO₂N(COCH₂)₂, -CO₂N(COCH₂)₂, - CO₂H -CHO, -CHOCH₂, -N=C=O, -SO₂CH=CH₂, -N(COCH)₂, -S-S-(C₅H₄N) may also be added to pendant chains of the collagen to covalently bond to itself (i.e., the collagen) or allow covalent bonding to occur with the glycosaminoglycans.

In embodiments, the collagen may be modified through the addition of an oxidizing agent. Contacting collagen with an oxidizing agent creates oxidative cleavage along portions of the collagen thereby creating pendant aldehyde groups capable of reacting with the glycosaminoglycans. The oxidizing agent may be, for example, iodine, peroxide, periodic acid, hydrogen peroxide, a periodate, a compound containing periodate, sodium periodate, a diisocyanate compound, a halogen, a compound containing halogen, n-bromosuccinimide, a permanganate, a compound containing permanganate, ozone, a compound containing ozone, chromic acid, sulfuryl chloride, a sulfoxide, a selenoxide, an oxidizing enzyme (oxidase), and combinations thereof. In embodiments, the oxidizing agent may be periodic acid.

Non-biodegradable materials may also be used to construct medical devices of the present disclosure. Suitable materials include fluorinated polymers (e.g., fluoroethylenes, such as polytetrafluoroethylene (PTFE), fluoropropylenes, and fluoroPEGs); polyolefins such as polypropylene, polyethylene and ultra high molecular weight polyethylene (UHMWPE); polyesters such as polyethylene terepththalate (PET); nylons; polyamides; polyurethanes; silicones; polybutesters; polyethylene glycol; polyaryletherketone; copolymers and combinations thereof. Additionally, non-biodegradable polymers and monomers may be combined with each other and may also be combined with other various biodegradable polymers and monomers to create a reactive implant.

In certain embodiments, implants according to the present disclosure may be constructed at least in part using shape memory polymers. Suitable polymers and monomers used to prepare hard and soft segments of shape memory polymers include caprolactones, dioxanones, lactides, glycolides, polyacrylates, polyamides, polysiloxanes, polyurethanes, polyether amides, polyurethane/ureas, polyether esters, urethane/butadiene copolymers, and combinations thereof.

In some embodiments, the implant may include metals (e.g., steel or titanium), metal alloys including degradable alloys such as iron-based or magnesium-based degradable alloys, and the like.

In certain embodiments, implants include porous substrates. The term "porous" as used herein may define openings and spacings which are present as a surface characteristic or a bulk material property, partially or completely penetrating the medical device. Pores may be created using methods within the purview of those skilled in the art, including but not limited to processes such as sintering, leaching of salt, sugar or starch crystals, and knitting or weaving of fibers. For example, a mesh having openings defined by a weave or knit pattern may be considered a porous material. Alternatively, a foam scaffold may be considered a porous material. Porous materials may have an open-cell structure, where the pores are connected to each other, forming an interconnected network. Conversely, porous substrates may be closed cell foams where the pores are not interconnected.

In certain embodiments, meshes such as composite meshes which have at least one substrate layer and one or more layers having a porous or non-porous construction may be used in forming a reactive implant. A non-porous layer may retard or prevent tissue ingrowth from surrounding tissues thereby acting as an adhesion barrier and preventing the formation of unwanted scar tissue. For example, a collagen composite mesh such as PARIETEX^{™} Composite Mesh (commercially available from Covidien) may be used in the reactive implant of the present disclosure. PARIETEX^{™} Composite mesh is a 3-dimensional polyester weave with a resorbable collagen film bonded on one side. Other meshes and devices which include collagen may be useful in certain embodiments of the present disclosure.

Other suitable meshes include those sold under the names PARIETENE®, PERMACOL™, PARIETEX™, SURGIPRO™, PRO-GRIP™ Self-fixating mesh, (all commercially available from Covidien); PROLENE™ (commercially available from Ethicon, Inc.); MARLEX®, DULEX®, 3D MAX® mesh, PERFIX® plug, VENTRALEX®, and KUGEL® patch (all commercially available from C.R. Bard, Inc.); COMPOSIX®, SEPRAMESH®, and VISILEX® (commercially available from Davol, Inc.); (DUALMESH®, MYCROMESH®, and INFINIT® mesh (all commercially available from W.L. Gore).

Medical devices used to create reactive implants of the present disclosure include, but are not limited to, meshes, patches, scaffolds, soft tissue repair devices, sutures, staples, tacks, grafts, tapes, gauzes, buttresses, pledgets, tissue engineering scaffolds, stents, tissue wraps, and combinations thereof.

A reactive component is combined with the medical device imparting reactive functional groups to the medical device surface, creating the reactive implant. Suitable reactive components may include crosslinkers, adhesives, sealants, couplers, and the like, which are functionalized with at least one free reactive group capable of bonding the medical device to tissue (or free amines). More specifically, reactive components include, but are not limited to, isocyanates, N-hydroxy succinimide ("NHS"), cyanoacrylates, aldehydes (e.g., formaldehydes, glutaraldehydes, glyceraldehydes, and dialdehydes), genipin, and other compounds possessing chemistries having some affinity for the substrate, tissue, or both. Reactive components of the present disclosure may also include any natural or synthetic crosslinker, including, but not limited to, aldehydes such as those listed above; diimides; diisocyanates; cyanamide; carbodiimides; dimethyl adipimidate; starches; and combinations thereof. The reactive components may be monofunctional, difunctional or multi-functional monomers, dimers, small molecules, or oligomers formed prior to or during implantation.

In one embodiment, the reactive component is genipin, as shown in Structure I.

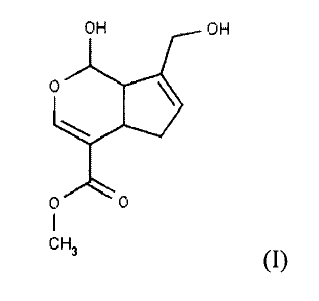

Genipin is an aglycone derived from an iridoid glycoside called geniposide present in the fruit of the Gardenia jasminoides Ellis. As used herein the term "genipin" includes, genipin, its derivatives, analogs, and any stereoisomer or mixture of stereoisomers of genipin. Genipin may be used as a natural crosslinker for amine-containing proteins including, but not limited to, collagen, elastin, gelatin, and chitosan. Because genipin is naturally occurring, it has low toxicity which may be useful for in situ medical applications. Genipin may be prepared by oxidation followed by reduction and hydrolysis or by enzymatic hydrolysis of the parent compound geniposide. Alternatively, racemic genipin may be prepared synthetically.

Genipin is colorless, but it reacts with primary free amines and subsequently undergoes oxidative polymerization, creating blue pigments. In embodiments, the blue color produced may darken as polymerization continues over the course of a few hours to several days. The blue pigments produced may provide an added benefit of a natural color identifier to visually locate the points of chemical attachment of the implant, such as a mesh. In other embodiments, the blue pigments formed during a chemical reaction may also be used to indicate to the user the degree of crosslinking. For example, a darker blue color (when crosslinking with amines) may indicate that more crosslinking has occurred as compared to a lighter blue color. Additionally, genipin may exhibit a specified degree of color change, associated with a specified degree of crosslinking. Additionally, the crosslinking may also increase the stiffness of the cross-linked area of the implant.

The intensity of the color may be concentration dependent, thus color gradients may be formed by varying the concentration of the genipin across a substrate or by crosslinking the substrate with the genipin at different rates. The crosslinking kinetics may also be expedited by controlling the pH of the genipin solution.

### Chemical means of attachment/interaction

The substrate is combined with a reactive component having at least one free reactive group capable of chemically bonding with living tissue. In certain embodiments, the reactive component may crosslink with itself around and/or throughout the substrate, while maintaining free reactive groups for crosslinking with a tissue surface. In other embodiments, the reactive component can crosslink with an amine-containing substrate or an amine-containing coating thereon and may further react to tissue. In alternate embodiments, a first reactive group of the reactive component can be used to chemically bond to the substrate and a second reactive group of the reactive component can be used to chemically bond the substrate to tissue.

In some embodiments, the reactive component can be immobilized to the substrate by reacting with itself. For example, the reactive component can react with itself, encapsulating the substrate, forming an intricate network encompassing the substrate, or portions thereof. In other embodiments, when the substrate is porous, the reactive component may penetrate interstices or pores, reaching the interior of the implant. In certain embodiments, the reactive component may not chemically bond to the substrate (if no free amines are present). The reactive component may also maintain free reactive groups for further reacting with tissue.

In alternate embodiments, the reactive component can be immobilized to the substrate by chemical bonding with the substrate. Thus, the reactive component may have more than two reactive groups. For example, a first reactive group of the reactive component may react with the substrate and a second reactive group may remain free for reacting with tissue. More than one reactive group may be free for reacting with tissue; in embodiments from about 1 reactive group to about 8 reactive groups may be free for reacting with tissue. For example, the reactive component may be reactive to a proteinaceous implant. The chemical reaction between the reactive component and the implant may bind the reactive component to the implant while leaving some reactive groups unreacted for future chemical reactions with a tissue surface in situ.

In embodiments, genipin may be applied as a reactive component for chemical bonding with a substrate. In other embodiments, genipin may be pre-mixed with an amine containing moiety and applied as a mixture for crosslinking on a surface of a substrate, thereby becoming adhered thereto.

### Physical means of attachment/interaction

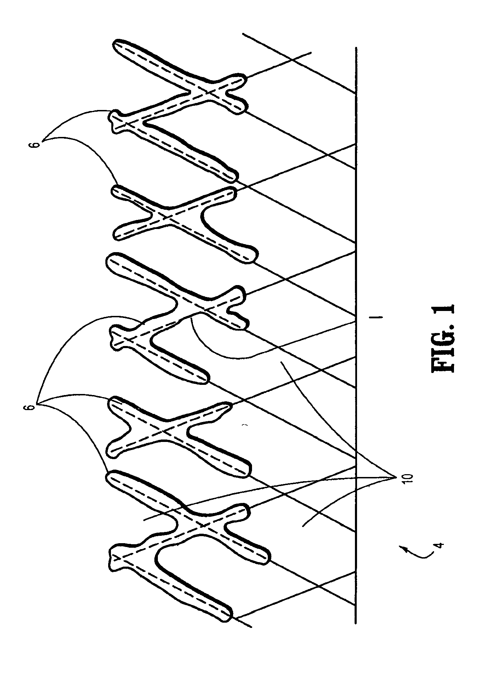

The reactive component may be supplied as a coating on the medical device, termed herein a "reactive coating." Figure 1 illustrates a mesh substrate 4, coated with a reactive coating 6. The mesh 4 includes pores 10 whereby the reactive coating does not fill the pores 10. However, it is envisioned that a reactive coating may wick into other porous substrates, penetrating and filling pores or interstices. The reactive coating 6 may reside on at least one side or at least a portion of the mesh 4 prior to sterilization and packaging. Methods for coating medical devices are within the purview of those skilled in the art, including but not limited to spraying, dipping, brushing, vapor deposition, co-extrusion, capillary wicking, film casting, molding and the like. The reactive component may be combined with the substrate in the form of a solution, film, foam, or powder on at least a portion of the substrate. The reactive component may be present in an amount of from about 0.001% by weight to about 10% by weight of the implant, in embodiments, from about 0.05% weight to about 5.0% weight of the reactive implant.

In embodiments, a substrate utilized to form an implant of the present disclosure possesses amine groups. Such amine groups may be found in the substrate itself, for example a collagen substrate, or may be included in a coating or layer on at least a portion of a surface of such substrate. These amine groups are capable of reacting with the reactive component described above, thereby forming crosslinked regions on the mesh where the reactive component and amine groups have reacted.

The crosslinked regions on the mesh provide increased mesh stiffness in the crosslinked regions in addition to increasing the overall stiffness of the mesh.

Alternatively, the reactive component may be immobilized to the substrate through mechanical interactions such as wicking into pores or capillary action. For example, with woven or knitted implants such as grafts or meshes, a genipin solution may be physically entrapped in pores or between fibers. It should be understood that other reactive solutions may also become physically entrapped in pores or between fibers. The implant may be further dried at a specified temperature and humidity level, removing residual solvent and leaving behind a reactive coating, creating a reactive implant.

Alternatively, the reactive coating may be applied to the device prior to implantation, for example soaking the medical device in a reactive solution in the operating room, prior to implantation.

For example, a reactive component may be supplied in a conduit to be used with a specialized injectable package material containing a substrate. Examples of an injectable package are disclosed in U.S. Patent Application Publication No. 2007/0170080 filed January 26, 2006, the entire disclosure of which is incorporated by reference herein. The reactive component may be injected into the substrate package any time prior to surgical use. In embodiments, the reactive component, water soluble or dispersible, saturates and swells the substrate in preparation for use. A bioactive agent may also be added either to the reactive component or directly into the substrate package at the time of use.

Upon reacting with amine-containing tissues, the reactive implant should fixate to tissue within a useful time range. In alternate embodiments, the crosslinker may be chemically "shielded" or "blocked" in aid of slowing the reaction with tissue, or the crosslinker of interest may simply have slow reaction kinetics.

The amount of time necessary for the reactive component to bind to the substrate and/or tissue may vary from about 1 second to about 6 hours, in embodiments about 30 seconds to about 2 hours.

Reactive components may be combined with solvents, including polar and nonpolar solvents to create solutions or coatings which will be applied to medical devices of the present disclosure. Non-limiting examples of solvents include water; saline; buffer salts; alcohols including methanol, ethanol, and propanol; dimethyl sulfoxide; dimethylformamide; chlorinated hydrocarbons such as methylene chloride, chloroform, and 1, 2-dichloro-ethane; and aliphatic hydrocarbons such as hexane, heptene, and ethyl acetate. Reactive components may be present in a solution from a concentration of about 0.1 % w/v to about 10% w/v.

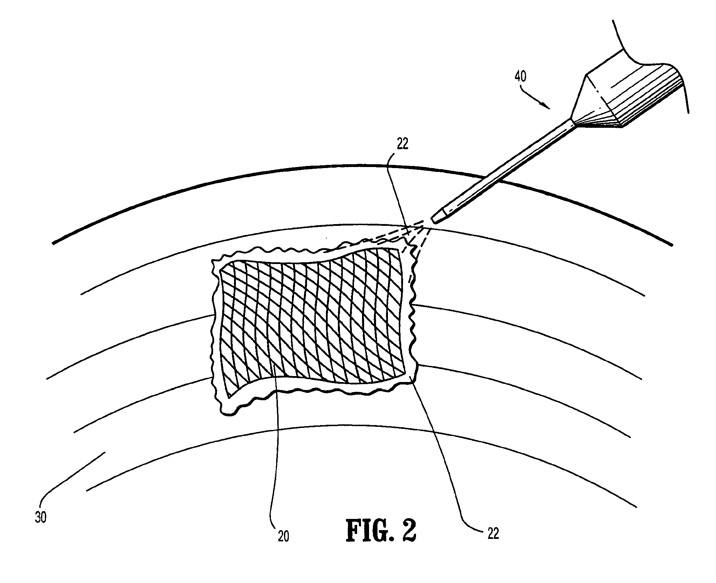

Figure 2 illustrates an alternate embodiment of a method by which the reactive implant may be created in situ. A reactive solution 22 is applied to an implant 20 in situ (creating a reactive implant), crosslinking the implant to a tissue surface 30. As shown, applicator 40 sprays the reactive solution 22 towards the implant 20, covering the implant 20. The reactive solution 22 crosslinks the implant 20 to the tissue surface 30, securing the implant 20 in situ. The reactive solution 22 covers the implant surface and may penetrate the mesh interstices (not shown). In certain embodiments, the reactive solution may be laparoscopically sprayed into the body. The reactive substrate may react with functional groups in tissue such as primary amine groups, secondary amine groups, hydroxyl groups, carboxylic groups, sulfonic groups and combinations thereof, and the like.

In certain embodiments, reactive implants of the present disclosure may further include a coating. Coatings may be applied to the implant for various reasons including increasing lubricity (for insertion), altering the chemical reactivity of the implant surface, promoting or preventing adhesions, or increasing biocompatibility. Coatings for implants may include any of the absorbable and non-absorbable materials listed above. In embodiments, the coating may include a polymer such as collagen. Collagen may, for example, help prevent any undesirable adhesion of the reactive coating with tissue in situ. Coatings may be applied to implants using any method within the purview of those skilled in the art including, but not limited to, dip coating, spray coating, solvent evaporation and the like.

In embodiments, the crosslinking or chemical bonding of a reactive component with a substrate results in a visible color change. For example, as described above, the crosslinking reaction of genipin with an amine-containing substrate causes blue pigments to form. This crosslinking reaction may be used to create a mark, pattern, or tattoo on a surface of the substrate. These marking may be used to aid in the observation, sizing, and placement of an implant within the body. In embodiments, a reactive component may be applied and crosslinked to one surface of an implant thereby providing an implant with at least one distinguishable surface. In embodiments, the markings may be used to identify which surface should be facing up and away from tissue and which surface should be facing down on the tissue upon implantation.

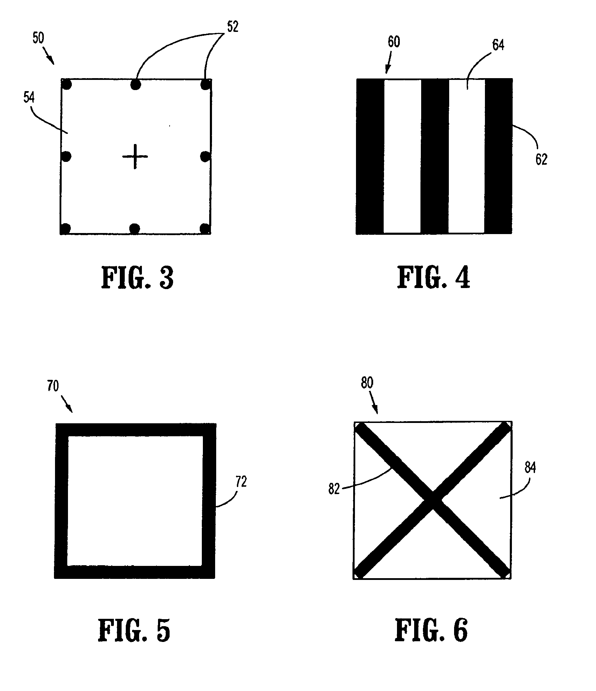

As illustrated in Figure 3, implant 50 may include crosslinked regions or markings 52 selectively distributed over a surface 54 of the implant 50. More specifically, the crosslinked regions are areas where the reactive component has crosslinked the amine functional groups on the mesh. The implant 50 includes a polyester mesh, having a collagen film attached to at least one surface of the mesh. The reactive component may be applied to the collagen surface 54 of the implant 50 to form markings 52 utilizing any of the methods described above. Similiarly, the reactive component may be applied to the mesh surface, which is then contacted with the collagen film/surface. In other embodiments, the reactive component may also be applied after the collagen attachment process. A variety of applicators within the purview of those skilled in the art may be used to apply the reactive component, including, for example, syringes, droppers, markers or pen-like applicators, brushes, sponges, patches, combinations thereof, and the like. The markings 52 may be in any shape and size to provide a visibly distinguishable mark or pattern to the surface 54 of the implant 50.

The reactive component may be applied to an amine functional surface of the mesh, i.e., collagen. The reactive component may be applied in solution or powder form, before, during, or after the collagen formation and attachment process.

In some embodiments, the markings 52 may be uniformly placed across the surface 54 of the implant 50 and evenly distributed and spaced to help the surgeon ascertain the size and placement of the implant 50 within tissue. The markings 52 may be calibrated against a scale-ruler to aid the surgeon in determining the size of the implant 50 that is needed (e.g., which areas may be trimmed or cut away, or if any additional pieces are needed to cover a defect). In other embodiments, the markings 52 may be placed at varying intervals across the surface 54 of the implant 50, such as having a more populated region of markings 52 on a portion of the surface 54 and a less populated region of markings 52 on other portions of the surface (not shown), thereby providing a distinctive pattern to help the surgeon determine the proper orientation of the implant 50 on tissue. In yet other embodiments, different markings may be utilized on a surface of an implant to help maintain the proper positioning and orientation of an implant during implantation.

Figure 4 illustrates an embodiment of an implant 60 which includes a series of crosslinked regions or markings 62 in the form of bands across the surface 64 of the implant 60. The markings 62 may be utilized to help determine the proper orientation and placement of the implant on tissue (e.g., the implant may be positioned with the bands vertically or horizontally placed across a tissue defect).

The crosslinking density of a reactive component with a substrate of the present disclosure may also be adjusted to provide stiffened regions within the implant. The concentration of reactive component may also be varied at different locations on the substrate, thus providing an implant with stiffened regions in those places where higher concentrations of the reactive component were located. The crosslinked regions will be more rigid than the uncross-linked regions and thus aid in the handling and unfolding of the implant once placed within the body while maintaining a desired flexibility. Thus, the crosslinked regions or markings 52, 62 on the substrates of Figures 3 and 4 may provide both visual and handling aids to the implants 50, 60. In other embodiments, the reactive component may only impart strength in localized regions of the substrate and does not induce a color change. Additionally, the crosslinking density may be adjusted by controlling the hydrolysis profile of a biodegradable substrate or by changing the pore size of a porous substrate.

As illustrated in Figure 5, a reactive component may be applied to the perimeter of an implant 70 to provide crosslinked regions 72 at the edges/periphery and/or fringes of the implant 70. The crosslinked regions 72 enhance the handling and unfolding characteristics of the implant 70 and also make the implant 70 more tear resistant and easier to suture or staple if fasteners are utilized to hold the implant 70 in place. Figure 6 illustrates an implant 80 including crosslinked regions 82 in the shape of an "x" across the surface 84 of the implant 80. The crosslinked regions 82 extend to the extremities of the implant 70 (e.g., the corners for implant 80) to ensure that the entire implant unfolds upon implantation. It is envisioned that the reactive component may be applied in any variety of patterns or shapes to provide crosslinked regions that impart the desired handling properties to the implant.

Implants of the present disclosure may be utilized in any surgical procedure in which their use is indicated. In embodiments, implants of the present disclosure may be used in inguinal and/or ventral hernia repair, utilizing both open (surgical) and laparoscopic repair techniques.

In some embodiments, at least one bioactive agent may be combined with the substrate or reactive component utilized to form an implant of the present disclosure, or both. The term "bioactive agent," as used herein, is used in its broadest sense and includes any substance or mixture of substances that have clinical use. A bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the medical device in any suitable form of matter, e.g., films, powders, liquids, gels, combinations thereof, and the like.

Non-limiting examples of classes of bioactive agents which may be utilized in accordance with the present disclosure include: anti-adhesives, antimicrobials, antiinfectives, anti-thrombotics, anti-spasmatics, analgesics, antipyretics, anesthetics, antiepileptics, antihistamines, anti-inflammatories, anti-proliferatives, cardiovascular drugs, diagnostic agents, chemo agents, telomerase inhibitors, polymer drugs including polyaspirin and polydiflunisal, anti-platelet drugs, platelet activating drugs, angiogenic agents, gene therapy agents, protein therapeutics, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Anti-adhesive agents can be used to prevent adhesions from forming between the implantable medical device and the surrounding tissues. In addition, anti-adhesive agents may be used to prevent adhesions from forming between the coated implantable medical device and the packaging material. Some examples of these agents include, but are not limited to, poly(vinyl pyrrolidone), carboxymethyl cellulose, hyaluronic acid, alginate, collagen, polyethylene glycol, polyethylene oxide, polypropylene glycol, poly vinyl alcohols, poly acrylic acid, styrene sulfonic acid, polyhydroxyethylmethylacrylate, (pHEMA) and phospholipid vinyls; acrylic polymers such as sodium polyacrylate, polyethylacrylate, and polyacrylamide, polypropylene oxide, phosphorylcholine functional acrylates and methacrylates; homopolymers and combinations thereof. As noted above, in embodiments the materials utilized to form the substrate may include these materials, and thus an implant including such a substrate may function as an anti-adhesion barrier without the inclusion of any additional bioactive agents.

Suitable antimicrobial agents which may be included as a bioactive agent of the present disclosure include triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil; nonoxynol 9; fusidic acid; cephalosporins; and combinations thereof. In addition, antimicrobial proteins and peptides, such as lactoferrin and lactoferricin B, and antimicrobial polysaccharides, such as fucans and derivatives thereof, may be included as a bioactive agent in the present disclosure.

Other bioactive agents which may be included as a bioactive agent in accordance with the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as vasodilators and nitroglycerin; alkaloids; analgesics; narcotics such as codeine, dihydrocodeinone, meperidine, and morphine; non-narcotics such as salicylates, aspirin, acetaminophen, and d-propoxyphene; opioid receptor antagonists such as naltrexone and naloxone; anti-cancer agents; anti-convulsants; anti-emetics; antihistamines; antiinflammatory agents such as hormonal agents, hydrocortisone, prednisolone, prednisone, non-hormonal agents, allopurinol, indomethacin, phenylbutazone and the like; polymer drugs (e.g., polydiflunisol, polyaspirin, and protein therapeutics); prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents include viruses and cells; peptides; polypeptides and proteins, as well as analogs, muteins, and active fragments thereof; immunoglobulins; antibodies; nanobodies; cytokines (e.g., lymphokines, monokines, chemokines); blood clotting factors; hemopoietic factors; interleukins (IL-2, IL-3, IL-4, IL-6); interferons (3-IFN, a-IFN and y-IFN); erythropoietin; nucleases; tumor necrosis factor; colony stimulating factors (e.g., GCSF, GM-CSF, MCSF); insulin; anti-tumor agents and tumor suppressors; blood proteins; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor and insulin-like growth factor); protein inhibitors; protein antagonists and protein agonists; nucleic acids such as antisense molecules, DNA, DNA intercalators, RNA, and RNAi; oligonucleotides; polynucleotides; and ribozymes.

In embodiments, the bioactive agent may be a local anesthetic, such as bupivacaine, ropivacaine, combinations thereof, and the like.

The following examples are being submitted to illustrate embodiments of the present disclosure. These examples are intended to be illustrative only and are not intended to limit the scope of the present disclosure. Also, parts and percentages are by weight unless otherwise indicated.

### EXAMPLES

The following non-limiting examples show possible surgical implants such as meshes, packaging considerations for combining the substrate and the reactive component for controlling the reaction kinetics, and optional bioactive agents that may be added to the substrate to impart additional characteristics to the implant.

### EXAMPLE 1

A solution including polyethylene glycol end-capped with an NHS ester in methylene chloride is prepared at a concentration of about 40 % w/v. A PARIETEX™ mesh from United States Surgical, is dip coated in the solution and dried in an oven overnight at ambient temperature, removing any remaining solvent. The mesh is then implanted in situ, and the reactive implant (mesh) will, in the presence of tissue having a presenting amine, crosslink the collagen mesh to tissue, thereby forming an interpenetrating network. The reaction may take up to 30 minutes to complete.

### EXAMPLE 2

A reactive component is supplied in a conduit to be used in concert with a specialized injectable package containing a graft substrate. The reactive component may be injected into the graft package immediately prior to surgical use. The reactive component, water soluble or dispersible, saturates and swells the graft substrate in preparation for use.

Upon removal from the provided package, the implant is designed to react within a specified time range. Optionally, the crosslinker may be chemically shielded or blocked by a water soluble coating or a coating including buffer salts to slow the reaction, enabling the surgeon to reposition the graft if required.

### EXAMPLE 3

A polyester mesh is provided and properly positioned in situ by a surgeon. Once positioned, a genipin solution at a concentration of about 60 mg/mL is laparoscopically sprayed in situ, covering the mesh. The genipin solution covers the mesh and the surrounding tissue, creating a reactive implant. Over the next few hours, the genipin solution crosslinks the mesh to tissue, fixating the mesh in place.

### EXAMPLE 4

A mesh, having a strip of collagen film applied to its surface along its periphery, is provided. A genipin solution is applied to the mesh, whereby the genipin crosslinks the amine groups found in the collagen film along the periphery of the mesh. The crosslinking of the genipin with the amine groups in the collagen strip enhances the handling and unfolding characteristics of the mesh and also makes the mesh more tear resistant and easier to suture or staple if fasteners are utilized to hold the mesh in place.

It will be understood that various modifications may be made to the embodiments disclosed herein. Therefore, the above description should not be construed as limiting, but merely as an exemplification of preferred embodiments. Those skilled in the art will envision other modifications within the scope and spirit of the present disclosure. Such modifications and variations are intended to come within the scope of the following claims.

## Claims

1. A medical device comprising:
a substrate comprising at least one synthetic component;
at least one natural material on at least a portion of a surface of the substrate; and
a reactive component selectively applied to at least a portion of the medical device,
wherein the reactive component reacts with the at least one natural material to form at least one crosslinked region on the surface of the substrate.

2. The medical device of claim 1, wherein the crosslinked regions include visible markings, preferably wherein the markings form a pattern on the surface of the substrate.

3. The medical device of any preceding claim, wherein the crosslinked regions increase stiffness to portions of the substrate to which they are applied.

4. The medical device of any preceding claim, wherein the crosslinked regions are disposed at a periphery of the surface of the substrate, and/or wherein the crosslinked regions extend to extremities of the substrate.

5. The medical device of any preceding claim, wherein the reactive component is selected from the group consisting of genipin, isocyanates, N-hydroxy succinimides, cyanoacrylates, aldehydes, diimides, cyanamide, carbodiimides, dimethyl adipimidate, starches, and combinations thereof, preferably wherein the reactive component comprises genipin.

6. The medical device of any preceding claim, wherein the at least one synthetic component includes polymers derived from lactides, glycolides, caprolactones, valerolactones, carbonates, dioxanones, 1,dioxepanones, ethylene glycols, ethylene oxides, esteramides, γ-hydroxyvalerates, β-hydroxypropionates, alpha-hydroxy acids, hydroxybutyrates, poly (ortho esters), hydroxy alkanoates, tyrosine carbonates, poly(imide carbonates), poly(imino carbonates), polyurethanes, polyanhydrides, polymer drugs, fluoroethylenes, fluoropropylenes, fluoropolyethylene glycols, polyolefins, polyesters, nylons, polyamides, silicones, polybutesters, polyaryletherketones, and combinations thereof.

7. The medical device of any preceding claim, wherein the at least one natural material comprises amine functional groups, preferably wherein the at least one natural material comprises collagen.

8. The medical device of any preceding claim, wherein the substrate is selected from the group consisting of sutures, staples, stents, meshes, tapes, gauzes, soft tissue repair devices, buttresses, bands, ribbons, grafts, scaffolds, wound dressings, and foams.

9. The medical device of claim 8, wherein the substrate comprises a mesh.

10. The medical device of any preceding claim, wherein the medical device further comprises a polymer coating.

11. The medical device of any preceding claim, wherein the medical device further comprises a bioactive agent.

12. A method of increasing the stiffness of a mesh, the method comprising:
providing a substrate comprising at least one synthetic component in combination with at least one natural material comprising amine groups on at least a portion of a surface of the substrate;
contacting the substrate with a genipin solution; and
allowing the natural material and genipin to crosslink thereby creating localized crosslinked regions on the surface of the substrate,
wherein the localized crosslinked regions increase stiffness of the mesh.

13. The method of claim 12, wherein the crosslinked regions are visually identifiable.

14. The method of claim 12 or claim 13, wherein the crosslinked regions are disposed at a periphery of the surface of the substrate.

15. The method of any of claims 12 to 14, wherein the at least one synthetic component includes polymers derived from lactides, glycolides, caprolactones, valerolactones, carbonates, dioxanones, 1,dioxepanones, ethylene glycols, ethylene oxides, esteramides, γ-hydroxyvalerates, β-hydroxypropionates, alpha-hydroxy acids, hydroxybutyrates, poly (ortho esters), hydroxy alkanoates, tyrosine carbonates, poly(imide carbonates), poly(imino carbonates), polyurethanes, polyanhydrides, polymer drugs, fluoroethylenes, fluoropropylenes, fluoropolyethylene glycols, polyolefins, polyesters, nylons, polyamides, silicones, polybutesters, polyaryletherketones, and combinations thereof.

16. A surgical mesh comprising:
a polyester substrate;
a collagen film on at least a portion of a surface of the polyester substrate; and
a reactive component comprising genipin selectively applied to at least a portion of the mesh,
wherein the genipin crosslinks the collagen, thereby increasing stiffness of the mesh.

17. The surgical mesh of claim 16, wherein the substrate comprises a woven fabric.
